Europäisches Patentamt

(19) European Patent Office

Office européen des brevets

(11) Publication number: **0 132 089 B1**

(12) **EUROPEAN PATENT SPECIFICATION**

(45) Date of publication of patent specification: **27.10.93**  (51) Int. Cl.5: **A61K 31/20**

(21) Application number: **84304610.3**

(22) Date of filing: **05.07.84**

(54) **Fatty acid compositions.**

(30) Priority: **14.07.83 GB 8319073**

(43) Date of publication of application:
**23.01.85 Bulletin 85/04**

(45) Publication of the grant of the patent:
**27.10.93 Bulletin 93/43**

(84) Designated Contracting States:
**AT BE CH DE FR GB IT LI LU NL SE**

(56) References cited:
**EP-A- 0 115 419**        **DE-A- 2 749 492**
**DE-A- 3 213 744**        **FR-A- 2 231 379**
**GB-A- 1 082 624**        **GB-A- 2 033 745**

**SURGERY; July 1970, vol. 68, no. 1, pp. 175-179**

**Diabetic Medicine, 1990, 7, pp. 319-323**

**British Journal of Dermatology (1989), 120, paper 623, pp. 1-15**

(73) Proprietor: **SCOTIA HOLDINGS PLC**
**Efamol House**
**Woodbridge Meadows**
**Guildford Surrey GU1 1BA(GB)**

(72) Inventor: **Horrobin, David Frederick**
**P.O. Box 10**
**Nun's Island Montreal, H3E 1J8(CA)**

(74) Representative: **Miller, Joseph et al**
**J. MILLER & CO.**
**34 Bedford Row,**
**Holborn**
**London WC1R 4JH (GB)**

## Description

The invention relates generally to fatty acids and their pharmaceutical and dietary uses and particularly to the use of certain fatty acids for the preparation of medicaments.

The n-6 or $\Omega$-6 essential fatty acids (EFA's) are required in the body for the structure of membranes in and around cells, being believed to be necessary in particular for maintaining normal flexibility, fluidity and permeability of such membranes. Certain members of the series also act as precursors of prostaglandins (PG's), short-lived regulating agents which modulate many aspects of cellular function.

The pathways of metabolism of the n-6 EFA's and the related n-3 EFA's sharing, it is believed, common enzymes in the two pathways, are:

$$
\begin{array}{llll}
& \underline{\text{n--6}} & & \underline{\text{n--3}} \\
\Delta^{9,12} & 18{:}2 & 18{:}3 & \Delta^{9,12,15} \\
& \downarrow \quad \Delta^{6}\text{desaturase} & \downarrow & \\
\Delta^{6,9,12} & 18{:}3 & 18{:}4 & \Delta^{6,9,12,15} \\
& \downarrow \quad \text{elongation} & \downarrow & \\
\Delta^{8,11,14} & 20{:}3 & 20{:}4 & \Delta^{8,11,14,17} \\
& \downarrow \quad \Delta^{5}\text{desaturase} & \downarrow & \\
\Delta^{5,8,11,14} & 20{:}4 & 20{:}5 & \Delta^{5,8,11,14,17} \\
& \downarrow \quad \text{elongation} & \downarrow & \\
\Delta^{7,10,13,16} & 22{:}4 & 22{:}5 & \Delta^{7,10,13,16,19} \\
& \downarrow \quad \Delta^{4}\text{desaturase} & \downarrow & \\
\Delta^{4,7,10,13,16} & 22{:}5 & 22{:}6 & \Delta^{4,\,7,10,13,16,19}
\end{array}
$$

The n-3 acids are metabolised preferentially and as a result plasma levels of 18:3 n-3 are low and 18:4 n-3 and 20:4 n-3 are in trace amounts only. In contrast the n-6 acids are all normally detectable, though 18:3 n-6 is at low levels, being apparently converted to 20:3 n-6 more rapidly than its relatively slow production from 18:2 n-6. The elongation stages in the metabolic pathways are much more rapid than the desaturations. The acids are in the natural all-cis configurations. In the n-6 series, commonly used names for the 18:2 and 18:3 (octadeca di- and tri-enoic); 20:3 and 20:4 (eicosa tri - and tetra-enoic); and 22:4 (decosatetraenoic) acids are linoleic acid, $\gamma$-linolenic acid (GLA), dihomo-$\gamma$-linolenic acid (DGLA), arachidonic acid (AA) and adrenic acid. In the n-3 series only $\alpha$-linolenic acid (18:3) is commonly referred to by a non-systematic name.

All the n-6 acids are found in plasma and in cell membranes in moderate amounts with the exception of $\gamma$-linolenic acid, which is present only in trace quantities because of rapid conversion to dihomo-$\gamma$-linolenic acid.

Considering dietary requirements, it is well known that linoleic acid for example cannot be made by the body and so must be taken in the diet. However it has been generally thought that the body can metabolise linoleic acid to all the other n-6 EFA's and therefore that provided linoleic acid intake is adequate, no lack of the other n-6 acids will be found.

In previous patent applications of the present inventor (for example published EP-A-0 003 407, EP-A-0 004 770, EP-A- 0 019 423) it has been pointed out that this is not so and that the first enzyme in the pathway, the $\Delta$-6 desaturase which converts the linoleic acid to $\gamma$-linolenic acid, is not fully effective in a variety of conditions. The administration of $\gamma$-linolenic acid or dihomo-$\gamma$-linolenic acid or both has been suggested in the above patent applications as a way to by-pass this block and has been successful in treating a variety of clinical conditions.

In further studies of the plasma and red blood cells of patients with various diseases the inventor has now found that levels of the $C_{22}$ n-6 acids are reduced from normal in a variety of conditions and the low levels observed are not or not fully corrected by the administration of linoleic acid or, more significantly, of $\gamma$-linolenic acid or dihomo-$\gamma$-linolenic acid, even in substantial amounts.

The basis for these observations of low $C_{22}$ n-6 acids is believed to be that in many instances there is an excess of 2-series PG production, either alone or coupled with defects of the elongation reaction converting 20:4 to 22:4 and/or the desaturation reaction converting 22:4 to 22:5. The inventor's previous patent applications have pointed out that one way of coping with excess 2-series PG production is selectively increasing the formation of 1-series PG's by, inter alia, ensuring a sufficient supply of $\gamma$-linolenic or dihomo-$\gamma$-linolenic acid. However, the severe deficiencies of $C_{22}$ n-6 acids that appear to be common in diseases are a new factor. The acids are normally present in all cell membranes, and in high concentration in tissues such as the brain, and a possible reason for the deficiency is excessive consumption of arachidonic acid in conversion to 2-series PG's. Thus whilst $C_{22}$ n-6 acids may be given alone , specific steps are preferably taken to restore 1-series/2-series PG balance, and thus reduce arachidonic acid consumption, by giving $\gamma$-linolenic acid and/or dihomo-$\gamma$-linolenic acid as well.

The more important of the $C_{22}$ n-6 acids is the 22:4 acid, because the 22:5 acid cannot be converted back to the 22:4 acid in the body and thus the 22:5 acid will be ineffective to restore lack of it, and also because the 22:4 acid, specifically, has recently been found to give rise to homo-2 series PG's in the same way as GLA gives rise to 1-series PG's and arachidonic acid to 2-series PG's. These homo-2 series PG's have as yet been little investigated but may have important functions, requiring administration of 22:4 acid when it is deficient. A failure in the conversion to the 22:5 acid will give a requirement for administration of that acid as well to restore full normality, but if conversion is simply inefficient 22:4 alone may suffice.

It is therefore proposed that in conditions in which deficits of the $C_{22}$ n-6 acids are demonstrated, 22:4, or 22:4 and 22:5 in combination, optionally with $\gamma$-linolenic acid and/or dihomo-$\gamma$-linolenic acid, should be administered. The invention therefore provides for the use of $\Delta$ $^{7,10,13,16}$-docosatetraenoic acid for the preparation of a therapeutic composition for remedying a deficiency in plasma or cell wall levels of said acid, and consequently improving the condition of patients suffering from diseases which are accompanied by such deficiencies, such as

wart viruses and other viral infections; premenstrual syndrome and benign breast disease; the dry eye syndrome, scleroderma, rheumatoid arthritis, Crohn's disease, ulcerative colitis, systemic lupus erythematosus, collagenosis, Sjögrens syndrome, psoriasis, asthma, eczema, and other forms of auto-immune and inflammatory disorders; male infertility; diabetes; or schizophrenia, alcoholism (including effects both of excess and of withdrawal) and other psychiatric disorders,

$\Delta$ $^{6,9,12,15}$-octadecatetraenoic acid and higher acids of the n-3 series being absent where the deficiency is presented by patients suffering from

viral infections, male infertility, schizophrenia, premenstrual syndrome, systemic lupus erythematosus, collagenosis, Sjögrens syndrome, or psoriasis.

In EP-A-0 115 419 compositions of such acids with 18:4 n-3 and higher acids of the n-3 series are disclosed, and the preparation of medicaments comprising such n-3 acids is accordingly not within the scope of the present invention for conditions to which EP-A-0 115 419 relates.

Natural or synthetic acids in dietary or pharmaceutical vehicles may be used, and the acids may be used as such or as pharmaceutically acceptable and physiologically equivalent derivatives as detailed herein. Equivalence is demonstrated by entry into the pathway quoted herein, as evidenced by effects corresponding to those of the acids themselves or their natural glyceride esters. Thus, indirect identification of useful derivatives is by their having the valuable effect in the body of the acid itself, but conversion can be shown directly by gas chromatographic analysis of concentrations in blood, body fat, or other tissue by standard techniques, for example those of Pelick et al. p.23 "Analysis of Lipids and Lipoproteins", Ed. Perkins, American Oil Chemists Society, Champaign, Illinois, U.S.A. Suitable derivatives include those given for $\gamma$-linolenic acid and dihomo-$\gamma$-linolenic acid later herein; thus reference to any of the acids herein, particularly in the claims, is to be taken as including reference to the acids when in the form of such derivatives.

The significance of the $\gamma$-linolenic acid and dihomo-$\gamma$-linolenic acid when included is to ensure inter alia that the natural (and irreversible) pathway through to arachidonic acid and onwards is, when its function is impaired rather than inoperative, well supplied with its starting material rather than having to compete for limited supplies with the other pathways, for example, entry of $\gamma$-linolenic acid or arachidonic acid into ester storage forms or into PG synthesis, as well as in respect of PG balance as already discussed.

Conditions Treated

The conditions in which the inventor has demonstrated substantial deficiences in the amount of 22:4 or 22:5 acids in plasma or cell membranes include: viral infections, especially with wart viruses; premenstrual syndrome and benign breast disease; the dry eye syndrome, scleroderma, rheumatoid arthritis, Crohn's disease, ulcerative colitis and other forms of auto-immune and inflammatory disorders; male infertility; diabetes; and psychiatric disorders including schizophrenia and alcoholism (including effects both of excess and of withdrawal). Other auto-immune and inflammatory disorders are referred to in Tables III and IV herein.

Sources of the Acids

Natural sources of 22:4 or 22:5 include adrenal glands (22:5) and kidneys (22:4) obtained from slaughter houses, and 22:4 in the fat of the American Snapping Turtle. Calmic's GB-A-896903 refers for example to 22:5 in ox adrenal phospholipid. preparations, proposed for use against atherosclerosis compare JP-A-5592316 referring to hypercholesteraemia though without discussion of the background with the understanding now available. No reference to 22:4 is made. The acids can be isolated from these sources by, for example, saponification under mild non-oxidising conditions followed by preparative gas liquid chromatography. Synthesis of the acids is difficult but not impossible and provides another source.

Doses

Suggested dose ranges of the 22:4 and 22:5 acids are 5mg to 50g per day, preferably 50mg to 1g per day.

Packs

If it is not desired to have compositions comprising different active materials together, packs may be prepared comprising the materials presented for separate, or part joint and part separate administration in the appropriate relative amounts, and use of such packs is within the purview of the invention.

Dietary Compositions

The invention is chiefly described in terms of pharmaceutical compositions, but it will be understood that the $\gamma$-linolenic and other acids, being in the nature of dietary supplements, could be incorporated in a dietary margarine or other foodstuffs; such foodstuffs, possibly containing the other active materials and generally referred to in this description as dietary or pharmaceutical compositions, are within the purview of the invention and thus of the terms pharmaceutical compositions and packs.

Veterinary Applications

It will be understood that where a disorder of a kind calling for treatment in animals arises, the invention whilst described primarily in terms of human medicine and treatment is equally applicable in the veterinary field.

Amounts of $\gamma$- and Dihomo-$\gamma$-Linolenic Acids

A preferred daily dosage for an adult (weight ca 75 kg) is from 0.05 to 0.1 up to 1, 2, 5 or even 10 g as required for $\gamma$-linolenic acid, or equivalent weight (calculated as $\gamma$-linolenic acid) of dihomo-$\gamma$-linolenic acid or physiologically functional derivative of either. Amounts in particular may be 0.1 to 1.0 g daily. Corresponding doses of Oenothera oil containing 8 to 10% of $\gamma$-linolenic acid, are easily calculated.

Forms and Sources of $\gamma$-Linolenic and Other Acids

Convenient physiologically functional derivatives of $\gamma$-linolenic acid and dihomo-$\gamma$-linolenic acid for use according to the invention, as with the 22:4 and 22:5 acids, include salts, amides, esters including glycerides and alkyl (e.g. $C_1$ to $C_4$) esters, and phospholipids.

4

If desired, pharmaceutical compositions may be produced for use in the invention by associating the natural or synthetic acids, as such or as derivatives, with an acceptable pharmaceutical vehicle. It is however at present convenient to incorporate at least the γ-linolenic acid into compositions in the form of an available oil having a high γ-linolenic acid content, hence references to "oil" herein.

At the present time known natural sources of oils having a high γ-linolenic acid content are few (there are no known natural sources of significant amounts of dihomo-γ-linolenic acid). One source of oils currently available is the seed of Evening Primose species such as Oenothera biennis L. and Oenothera lamarckiana, the oil extract therefrom containing γ-linolenic acid (about 8%) and linoleic acid (about 72%) in the form of their glycerides together with other glycerides (percentages based on total fatty acids). Other sources of γ-linolenic acids are Borage species such as Borago officinalis which, though current yield per acre is low, provide a richer source of γ-linolenic acid than Oenothera oil. Recent studies on fungi which can be cultivated by fermentation promise a fungal oil source.

The seed oil extracts referred to above can be used as such or can for example if desired be fractionated to yield an oily composition containing the triglycerides of γ-linolenic and linoleic as the main fatty acid components, the γ-linolenic acid content being if desired a major proportion. Seed oil extracts appear to have a stabilising effect upon dihomoy-γ-linolenic acid if present.

## PHARMACEUTICAL PRESENTATION

The compositions according to the invention are conveniently in a form suitable for oral, rectal, parenteral or topical administration in a suitable pharmaceutical vehicle, as discussed in detail for example in Williams GB-A-1 082 624, to which reference may be made, and in any case very well known generally for any particular kind of preparation. Thus for example tablets, capsules, ingestible liquid or powder preparations, creams and lotions for topical application, or suppositories, can be prepared as required. Injectable solutions of hydrolysed Oenothera oil may be prepared using albumin to solubilise the free acid.

Advantageously a preservative is incorporated into the preparations. α-Tocopherol in concentration of about 0.1% by weight has been found suitable for the purpose.

It will be understood that the absolute quantity of active materials present in any dosage unit should not exceed that appropriate to the rate and manner of administration to be employed but on the other hand should also desirably be adequate to allow the desired rate of administration to be achieved by a small number of doses. The rate of administration will moreover depend on the precise pharmacological action desired.

The following Examples serve to illustrate pharmaceutical compositions useful in treatment according to the invention.

## EXAMPLES

Pharmaceutical compositions containing unit doses of one or more of the active materials of the present invention optionally with the oil of the seed of Oenothera biennis L. and optionally further for example with methyl dihomo-γ-linolenate, are presented by encapsulation in loft gelatine capsules by conventional methods.

The Oenothera oil is extracted from the seeds by one of the conventional methods of extraction such as cold pressure, screw pressure after partially cooking the seed, or solvent extraction.

Fractionation of a typical sample of this oil in the form of methyl esters shows the relative proportions:

| | |
|---|---|
| Palmitate | 6.15 |
| Stearate | 1.6 |
| Oleate | 10.15 |
| Linoleate | 72.6 |
| γ-Linolenate | 8.9 |

As preservative, α-tocopherol is added to the oil in a concentration 0.1%.

The following are specific examples of capsules, Examples 1, 2 and 4 optionally also containing 0.5g Oenothera oil, that may be given in the treatment of the conditions listed earlier, 1 to 8 per day (0.5g oil = ca 0. 045g γ-linolenic acid)

EXAMPLES

1. Capsules containing 75 or 100mg 22:4
2. Capsules containing 50mg 22:4 and 50mg 22:5
3. Capsules containing 50mg 18:3, in purified form or as in Oenothera oil, and 50mg 22:4
4. Capsules containing 50mg of 20:3 and 50mg of 22:4

Specifically, the capsules may be given against the conditions in respect of which the following tables show normal and diseased n-6 acid levels.

TABLE I.    Levels of fatty acids in the plasma phospholipids of control individuals (n,50) and women with cyclical (premenstrual) breast disease (n,21).  Levels are expressed as percentages of the total fatty acids present.  Each figure represents the mean $\pm$ SEM.

| Fatty Acid | Control | Cyclical breast Disease |
|---|---|---|
| 18:2n-6 | 21.45 $\pm$ 0.84 | 23.26 $\pm$ 0.90 |
| 20:3n-6 | 3.06 $\pm$ 0.09 | 2.43 $\pm$ 0.11 |
| 20:4n-6 | 11.36 $\pm$ 0.24 | 8.10 $\pm$ 0.48 |
| 22:4n-6 | 0.73 $\pm$ 0.04 | 0.23 $\pm$ 0.05 |
| 22:5n-6 | 1.12 $\pm$ 0.09 | 0.54 $\pm$ 0.07 |

Corresponding figures for non-cyclical (benign) breast disease, where the low $C_{22}$ n-6 levels are coupled with low n-3 levels also, are:

22.33 ± 0.89
2.54 ± 0.14
7.69 ± 0.36
0.33 ± 0.05
0.73 ± 0.09

TABLE II   As Table I. women with premenstrual syndrome signs and symptoms of mood change, fluid retention, weight gain, breast swelling and tenderness, and abdominal distension "Foll" (follicular phase 7-13 days post) "Lut" (late luteal phase, up to 7 days pre).  Each figure represents in this and the following Tables the mean $\pm$ standard deviation (SD).

| Fatty acid | Normal | Premenstrual Foll. | Premenstrual Lut. |
|---|---|---|---|
| 18:2n-6 | 22.74 $\pm$ 2.81 | 25.44 $\pm$ 3.46 | 24.87 $\pm$ 3.40 |
| 20:3n-6 | 3.06 $\pm$ 0.60 | 2.79 $\pm$ 0.64 | 2.93 $\pm$ 0.44 |
| 20:4n-6 | 11.36 $\pm$ 1.67 | 9.34 $\pm$ 1.40 | 9.26 $\pm$ 1.45 |
| 22:4n-6 | 0.73 $\pm$ 0.26 | 0.53 $\pm$ 0.33 | 0.48 $\pm$ 0.33 |
| 22:5n-6 | 1.12 $\pm$ 0.67 | 0.36 $\pm$ 0.81 | 0.16 $\pm$ 0.35 |

6

## TABLE III

| Fatty acid | 18:2n-6 | 18:3n-6 | 20:3n-6 | 20:4n-6 | 22:4n-6 | 22:5n-6 |
|---|---|---|---|---|---|---|
| **A. Red blood cell total phospholipids** | | | | | | |
| Normal + SD | 9.78 ± 1.64 | - | 1.37 ± 0.37 | 15.13 ± 1.98 | 5.54 ± 1.37 | 3.99 ± 1.85 |
| SLE * | 15.65 ± 3.84 | - | 1.91 ± 0.37 | 14.88 ± 3.43 | 2.11 ± 0.65 | 0.27 ± 0.17 |
| Collagenosis | - | - | - | - | - | - |
| Rheumatoid arthritis | 13.30 ± 2.09 | - | 1.96 ± 0.44 | 13.21 ± 1.60 | 2.23 ± 0.59 | 0.35 ± 0.17 |
| Sjögren's Syndrome | 13.69 ± 2.68 | - | 1.69 ± 0.24 | 13.69 ± 3.03 | 2.14 ± 0.65 | 0.24 ± 0.19 |
| **B. Plasma total phospholipids** | | | | | | |
| Normal + SD | 21.45 ± 2.81 | 0.16 ± 0.12 | 3.06 ± 0.60 | 11.36 ± 1.67 | 0.73 ± 0.26 | 1.12 ± 0.67 |
| SLE * | 23.13 ± 4.72 | - | 2.65 ± 0.44 | 11.14 ± 2.47 | 0.36 ± 0.21 | 0.09 ± 0.11 |
| Collagenosis | 22.19 ± 2.88 | 0.05 ± 0.10 | 3.95 ± 0.58 | 8.18 ± 1.48 | 0.33 ± 0.19 | 0.15 ± 0.11 |
| Rheumatoid arthritis | 22.19 ± 2.74 | - | 2.84 ± 0.56 | 10.62 ± 1.88 | 0.40 ± 0.32 | nd |
| Sjögren's Syndrome | 23.81 ± 2.35 | - | 2.86 ± 0.71 | 10.96 ± 1.17 | 0.19 ± 0.20 | 0.06 ± 0.14 |

* Systemic Lupus Erythematosus

nd = not detected

## TABLE IV

Plasma Phospholipids

| Fatty Acid | Normal | Psoriasis |
|---|---|---|
| 18:2n-6 | 21.45 + 2.81 | 22.66 + 5.79 |
| 18:3n-6 | 0.16 + 0.12 | 0.05 + 0.09 |
| 20:3n-6 | 3.06 + 0.60 | 2.80 + 0.80 |
| 20:4n-6 | 11.36 + 1.67 | 8.95 + 1.44 |
| 22:4n-6 | 0.73 + 0.26 | 0.35 + 0.32 |
| 22:5n-6 | 1.12 + 0.67 | 0.15 + 0.14 |

| Asthma *' | Eczema *' |
|---|---|
| 21.99 + 3.31 | 29.50 + 3.42 |
| 0.22 + 0.31 | nd |
| 2.96 + 0.55 | 2.63 + 0.52 |
| 9.18 + 1.39 | 6.75 + 1.12 |
| 0.14 + 0.22 | 0.38 + 0.24 |
| nd | 0.13 + 0.29 |

*' Coupled with low n-3 acid levels.

| Male Infertility | Diabetes |
|---|---|
| 22.6 ± 2.75 | 23.6 ± 2.94 |
| 0.13 ± 0.08 | 0.08 ± 0.05 |
| 3.80 ± 0.96 | 2.64 ± 0.71 |
| 9.56 ± 1.52 | 9.36 ± 1.24 |
| 0.32 ± 0.34 | 0.42 ± 0.31 |
| nd | 0.38 ± 0.24 |

The method of determination of acid levels was that plasma samples (1 ml) were extracted with chloroform:methanol (2:1). The extract was filtered through sodium sulphate, evaporated to dryness, and taken up in 0.5 ml chloroform:methanol. The lipid fractions were separated by thin layer chromatography on silica gel plates. The phospholipid fraction, taken to reflect essential fatty acid changes most sensitively, was methylated using boron trifluoride-methanol. The resulting methyl esters of the fatty acids were separated and measured using a Hewlett Packard 5880 gas chromatograph with a six foot column packed with 10% silar on chromosorb WAW 106/230. The carrier gas was helium (30 ml/min). Oven temperature was programmed to rise from 165°C to 190°C at 2°C/min. Detector temperature was 220°C and injector temperature 200°C. Retention times and peak areas were automatically computed by Hewlett Packard Level 4 integrator. Peaks were identified by comparison with standard fatty acid methyl esters.

## Claims

1. The use of $\Delta^{7,10,13,16}$-docosatetraenoic acid for the preparation of a therapeutic composition for remedying a deficiency in plasma or cell wall levels of said acid, and consequently improving the condition of patients suffering from diseases which are accompanied by such deficiencies, such as

   wart viruses and other viral infections; premenstrual syndrome and benign breast disease; the dry eye syndrome, scleroderma, rheumatoid arthritis, Crohn's disease, ulcerative colitis, systemic lupus erythematosus, collagenosis, Sjögrens syndrome, psoriasis, asthma, eczema, and other forms of auto-immune and inflammatory disorders; male infertility; diabetes; or schizophrenia, alcoholism (including effects both of excess and of withdrawal) and other psychiatric disorders,

   $\Delta^{6,9,12,15}$-octadecatetraenoic acid and higher acids of the n-3 series being absent where the deficiency is presented by patients suffering from

   viral infections, male infertility, schizophrenia, premenstrual syndrome, systemic lupus erythematosus, collagenosis, Sjögrens syndrome, or psoriasis.

2. The use set out in claim 1, where the therapeutic composition also comprises $\gamma$-linolenic acid or dihomo-$\gamma$-linolenic acid.

3. The use set out in claim 2, where the therapeutic composition also comprises $\Delta^{4,7,10,13,16}$-docosapentaenoic acid.

4. The use set out in claim 2, where the therapeutic composition is presented in dosage units of said docosaenoic acid(s) of 5 mg to 50 g, preferably 50 mg to 1 g, per day.

5. The use set out in claim 2, where the therapeutic composition is presented in dosage units of said linolenic acid(s) of 50 mg to 10 g, preferably 100 mg to 1 g, per day.

## Patentansprüche

1. Die Benutzung von $\Delta$ 7,10,13,16 docosatretraeonic Säure für die Herstellung einer therapeutischen Zusammensetzung zum Abstellen eines Mangels an Plasma oder Zellwandspiegeln der besagten Säure und somit zur Verbesserung des Zustandes von Patienten, welche an Kranheiten leiden, die mit solchan Mängeln einhergehen, wie z.B.

   Warzenviren und andere Virusinfektionen; prämenstruelles Snydrom und gutartige Brusterkrankung; das Trockenaugen-Syndrome, Sklerodermie, crohnsche Krankheit, ulzerative Kolitis, erythematöser Körperlupus, Kollagenose, rheumatische Arthritis, Sjögrens-Syndrom, Psoriasis, Asthma, Ekzem und andere Formen von auto-immunen und entzündlichen Störungen; männliche Unfruchtbarkeit; Diabetes oder Schizophrenie, Alkoholismus (Auswirkungen sowohl des Übermaßes als auch der Entziehung inbegriffen und andere psychiatrische Störungen.

   Wo $\Delta$-6,9,12,15 octadecatetraeonic Säure und höhere Säuren der n-3 Reihe fehlen, wird der Mangel durch Patienten aufgezeigt, die an Folgendem leiden:

   Virusinfektionen, männlicher Unfruchtbarkeit, Schizophrenie, prämenstruellem Syndrom, erythematösem Körperlupus, Kollagenose, Sjögrens-Syndrom oder Psoriasis.

2. Die Benutzung gemäß Anspruch 1, dadurch gekennzeichnet, daß die therapeutische Zusammensetzung ebenfalls $\gamma$-Linolensäure oder Dihomo-$\gamma$-Linolensäure einbezieht.

3. Die Banutzung gemäß Anspruch 2, dadurch gekennzeichnet, daß die therapeutische Zusammensetzung ebenfalls $\Delta$-4,7,10,13,16 docosapentaeonic Säure einbezieht.

4. Die Benutzung gemäß Anspruch 2, dadurch gekennzeichnet, daß die therapeutische Zusammensetzung in Dosierungseinheiten der besagtan docosaenoic Säure(n) von 5 mg bis 50 g, möglichst 50 mg bis 1 g, pro Tag bereitgestellt wird.

**5.** Die Benutzung nach Anspruch 2, dadurch gekennzeichnet, daß die therapeutische Zusammensetzung in Dosierungseinheiten der besagten Linolensäure(n) von 50 mg bit 10, möglichst 100 mg bis 1 g, pro Tag bereitgestellt wird.

**Revendications**

**1.** Utilisation de l'acide $\Delta^{7,10,13,16}$-docosatétraénoïque pour préparer une composition thérapeutique destinée à remédier à un taux insuffisant de cet acide dans le plasma ou les parois cellulaires, et, en conséquence, à améliorer l'état de patients souffrant de pathologies accompagnées par ces déficiences, telles que

les virus de la verrue humaine et d'autres infections virales; le syndrome pré-menstruel et les maladies bénignes des seins ; le syndrome de l'oeil sec, la sclérodermie, l'iléite régionale, la recto-colite hémorragique, le lupus érythémateux aigu disséminé, la collagénose, l'arthrite rhumatoïde, le syndrome de Sjögrens, le psoriasis, l'asthme, l'eczéma et d'autres formes de troubles autoimmunes et inflammatoires ; la stérilité masculine ; le diabète ; ou encore la schizophrénie, l'alcoolisme (qui englobe les effets tant d'un excès que d'un arrêt) et les autres troubles spychiatriques, en l'absence d'acide $\Delta^{6,9,12,15}$-octadécatétraénoïque et d'acides supérieurs de la série n-3, la déficience étant présentée par des patients souffrant

d'infections virales, de stérilité masculine, de schizophrénie, d'un syndrome pré-menstruel, de lupus érythémateux aigu disséminé, de collagénose, du syndrome de Sjögrens ou d'un psoriasis.

**2.** Utilisation selon la revendication 1, dans laquelle la composition thérapeutique comprend aussi de l'acide γ-linolénique ou de l'acide dihomo-γ-linolénique.

**3.** Utilisation selon la revendication 2, dans laquelle la composition thérapeutique comprend aussi de l'acide $\Delta^{4,7,10,13,16}$-docosapentaénoïque.

**4.** Utilisation selon la revendication 2, dans laquelle la composition thérapeutique est présentée en unités posologiques dudit ou desdits acides docosaénoïques de 5 mg à 50 g, de préférence de 50 mg à 1 g par jour.

**5.** Utilisation selon la revendication 2, dans laquelle la composition thérapeutique est présentée en unité posologique dudit ou desdits acides linoléniques de 50 mg à 10 g, et de préférence de 100 mg à 1 g par jour.